(19) Europäisches Patentamt
European Patent Office
Office européen des brevets

(11) Veröffentlichungsnummer: **0 013 361**
**B1**

(12) # EUROPÄISCHE PATENTSCHRIFT

(45) Veröffentlichungstag der Patentschrift: **16.09.81**

(21) Anmeldenummer: **79105053.7**

(22) Anmeldetag: **10.12.79**

(51) Int. Cl.³: **C 07 C 103/76,** C 07 C 103/78, C 07 C 125/067, C 07 C 155/02, C 07 D 521/00 // C07D307/68, C07D333/38, C07D213/81, C07D261/18, C07D263/34, C07D249/08, C07D401/12, (C07D401/12, 233/66, 213/18)

(54) **Substituierte N-Halogenmethylanilide und Verfahren zu ihrer Herstellung und Verwendung.**

(30) Priorität: **18.12.78 DE 2854599**

(43) Veröffentlichungstag der Anmeldung: **23.07.80 Patentblatt 80/15**

(45) Bekanntmachung des Hinweises auf die Patenterteilung: **16.09.81 Patentblatt 81/37**

(84) Benannte Vertragsstaaten: **AT BE CH DE FR GB IT LU NL SE**

(56) Entgegenhaltungen:
**EP-A-0 005 591**
**DE-B-1 153 756**
**DE-A-1 542 956**
**DE-A-2 119 518**
**DE-A-2 134 173**
**DE-A-2 147 260**
**DE-A-2 648 008**
**DE-A-2 704 281**
**DE-A-2 742 583**
**DE-A-2 813 335**
**US-A-3 637 847**

(73) Patentinhaber: **BASF Aktiengesellschaft, Carl-Bosch-Strasse 38, D-6700 Ludwigshafen (DE)**

(72) Erfinder: **Plath, Peter, Dr. Dipl.-Chem., Berner Weg 24, D-6700 Ludwigshafen (DE)**
Erfinder: **Eicken, Karl, Dr. Dipl.-Chem., Waldstrasse 63, D-6706 Wachenheim (DE)**
Erfinder: **Rohr, Wolfgang, Dr. Dipl.-Chem., Gontardstrasse 4, D-6800 Mannheim 1 (DE)**

**EP 0 013 361 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Substituierte N-Halogenmethylanilide und Verfahren zu ihrer Herstellung und Verwendung

Die Erfindung betrifft neue wertvolle N-Halogenmethyl-carbonsäureanilide, ein Verfahren zu ihrer Herstellung und ihre Verwendung zur Herstellung neuer wertvoller N-Carbonsäureanilide.

Es ist bekannt, durch Addition von Säurechloriden wie Chloressigsäurechlorid, Cyclopropancarbonsäurechlorid, Methoxyessigsäurechlorid oder 2,4-Dichlorbenzoylchlorid aus Schiffsche Basen wie 2,6-Diäthyl-N-methylenanilin oder 2-Methylen-6-tert.-butyl-N-methylenanilin die entsprechenden N-Chlormethyl-carbonsäureanilide herzustellen, die wertvolle Zwischenprodukte für die Herstellung von bekannten Wirkstoffen von Herbiziden oder Insektiziden sind. (US-PS 3 714 299, 3 810 981, 3 630 716 und 3 637 847, ferner DE-OS 1 542 950).

Es sind ferner N-Aryl-N-chlormethylcarbamate durch Umsetzung von Paraformaldehyd, Thionylchlorid und N-Arylcarbamaten in bekannter Weise erhalten worden (DE-OS 2 119 518), wobei in der zitierten Offenlegungsschrift betont wird, dass derartige Produkte nur nach den dort offenbarten Verfahren, nicht jedoch durch Addition von Chlorkohlensäure-O-arylestern an trimerisierte Schiffsche Basen [Hexahydrotriazine] erhältlich seien.

Es ist ferner bekannt, N-Azolylmethyl-chloracetanilide als Fungizide zu verwenden (DE-A-26 48 008, DE-A-27 04 281). Ihre Wirkungen befriedigen jedoch nicht. Demgegenüber haben die unter Verwendung der neuen N-Halogenmethylcarbonsäureanilide herstellbaren N-Azolylmethylcarbonsäureanilide überraschend eine bessere fungizide Wirkung.

Obwohl die Addition beispielsweise von Benzoylchlorid an N-Methylen-2,6-dimethylanilin in den US-Patentschriften 3 630 716 und 3 637 847 beansprucht wird, ist in der Literatur nichts über den dabei zu erhaltenden Stoff N-Chlormethyl-2,6-dimethyl-N-benzoylanilin zu finden. Ähnlich verhält es sich mit weiteren N-Chlormethyl-carbonsäureaniliden, die zwar beansprucht, nicht jedoch durch Herstellungsbeispiele oder Ausführungen in der Beschreibung belegt sind.

Es wurde nun gefunden, dass Verbindungen der allgemeinen Formel

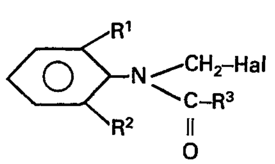

worin
$R^1$ Methyl, Äthyl, Isopropyl, Methoxy oder Chlor,
$R^2$ Methyl, Äthyl, Isopropyl oder Chlor
$R^3$ gegebenenfalls durch Halogen substituiertes Benzyl oder 1-Phenyläthyl,
oder gegebenenfalls ein- oder zweifach durch Methyl, Fluor, Chlor, Brom, Jod, Cyan, Trifluormethyl, Nitro, Methoxy oder Methylthio substituiertes Phenyl,
oder einen gegebenenfalls durch Methyl oder Chlor substituierten 5- oder 6-gliedrigen heterocyclischen Rest mit einem oder zwei gleichen oder verschiedenen Heteroatomen aus der Gruppe Sauerstoff, Schwefel und Stickstoff
oder den Rest $-CH_2-Y$ oder $-\overset{CH_3}{\overset{|}{C}H}-Y$ bedeutet, wobei Y ein $C_1$–$C_4$-Alkylthiol oder ein gegebenenfalls ein- oder mehrfach durch Chlor, Fluor, Methyl, Trifluormethyl oder Nitro substituiertes Phenoxyl oder Phenylthiol bedeutet,
oder $R^3$ den Rest $-X-R^4$ bedeutet,
wobei X Sauerstoff oder Schwefel,
$R^4$ ein gegebenenfalls ein- oder zweifach durch Fluor, Chlor, Brom, Trifluormethyl, $C_1$–$C_4$-Alkyl, Nitro, Methoxy oder Methylthio substituiertes Phenyl bedeutet, und Hal Fluor, Chlor oder Brom bedeutet, wertvolle Zwischenprodukte für die Herstellung wertvoller neuer N-Carbonsäureanilide sind.

Es wurde ferner gefunden, dass man die N-Halogenmethyl-N-arylcarbonsäureanilide erhält, wenn man ein substituiertes N-Methylenanilin der Formel

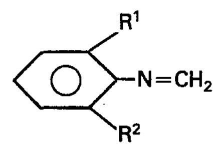

mit einem Carbonsäurehalogenid der Formel $R^3$–CO–Hal in einem Lösungsmittel umsetzt, wobei Hal, $R^1$, $R^2$ und $R^3$ die oben genannten Bedeutungen haben. Diese Umsetzung steht im Widerspruch zu der in DE-OS 2 119 518 offenbarten Lehre.

Ferner wurde gefunden, dass man Verbindungen der Formel

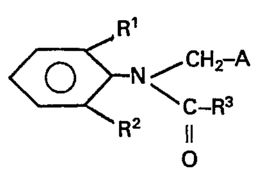

worin $R^1$, $R^2$ und $R^3$ die oben angegebenen Bedeutungen haben und A ein gegebenenfalls ein- oder mehrfach durch Methyl, Äthyl, Isopropyl, Chlor, Brom oder Nitro substituiertes Pyrazol, Imidazol oder 1,2,4-Triazol bedeutet, erhält, wenn man ein N-Halogenmethylcarbonsäureanilid der Formel

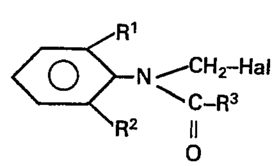

in der $R^1$, $R^2$, $R^3$ und Hal die oben genannten Bedeutungen haben, mit einem Azol der Formel

A–M, in welcher A die oben angegebene Bedeutung hat und M Wasserstoff, Kalium oder Natrium bedeutet, gegebenenfalls in Gegenwart eines Verdünnungsmittels oder eines Säurebindemittels oder beiden umsetzt.

Die Herstellung der neuen Verbindung kann wie folgt vorgenommen werden:
Verwendet man 2,6-Dimethyl-N-methylenanilin und 2-Furoylchlorid als Ausgangsmaterialien, dann kann der Reaktionsablauf durch folgendes Formelschema veranschaulicht werden:

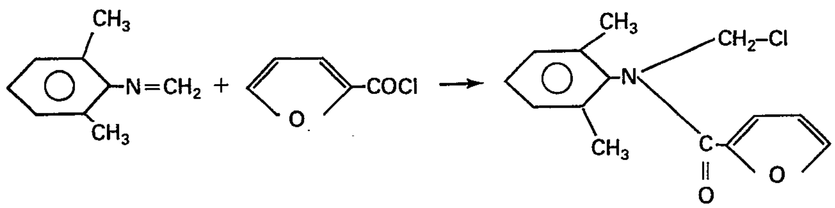

In den Formeln bedeutet $R^1$, $R^2$ vorzugsweise Methyl, Äthyl oder Isopropyl,
und $R^3$ bevorzugt Phenyl, Benzyl, 1-Phenyläthyl, 2-Chlor-α-phenyläthyl, 2-Methylphenyl, 3-Methylphenyl, 4-Methylphenyl, 2-Chlorphenyl, 3-Chlorphenyl, 4-Chlorphenyl, 2-Fluorphenyl, 3-Fluorphenyl, 4-Fluorphenyl, 3-Bromphenyl, 2-Jodphenyl, 2,6-Fluorphenyl, 3-Cyanphenyl, 2-Nitrophenyl, 3-Nitrophenyl, 4-Nitrophenyl, 3-Trifluormethylphenyl, 4-Trifluormethylphenyl, 3-Methoxyphenyl, 4-Methylthiophenyl, 2,3-Dichlorphenyl, 2,4-Dichlorphenyl, Methylthiomethyl, Phenylthio-methyl, Phenoxymethyl, 2,4-Dichlorphenoxymethyl, Thienyl-2-, Thienyl-3, Furyl-2, Furyl-3, 2,5-Dimethylfuryl-3, 4-Chlorthienyl-3, 2-Chlorpyridyl-3, 2,6-Dichlorpyridyl-4, 3-Methylisozolyl-5, 4-Methyl-oxazolyl-5, Phenoxy, 2,4-Dichlorphenoxy, 2,3-Dichlorphenoxy, 4-Nitrophenoxy, 3,5-Dimethoxy-phenoxy, 4-Isopropylphenoxy, 4-tert.-Butyl-phenoxy, 3-Methylphenoxy, 4-Methylthiophenoxy.

Die als Ausgangsstoffe zu verwendenden Schiffschen Basen lassen sich nach bekannten Methoden (vgl. US 3 637 847) aus Paraformaldehyd und Anilinen in Toluol herstellen. Die ebenfalls als Ausgangsmaterialien benötigten Carbonsäurechloride sind entweder bekannte Laborchemikalien oder sind nach Methoden synthetisierbar, die aus der Literatur bekannt sind. Die Azole A–M (mit M=H) sind gebräuchliche und allgemein bekannte Verbindungen der organischen Chemie.

Für die erfindungsgemässen Umsetzungen kommen als Verdünnungsmittel vorzugsweise Toluol, Ligroin oder n-Hexan in Frage. Zur Herstellung der Azol- oder Cyanamid-Verbindungen können jedoch auch Lösungsmittel wie Acetonitril oder Dimethylformamid verwendet werden.

Als Säurebindemittel können anorganische Säureakzeptoren, beispielsweise Alkalicarbonat, beispielsweise Natriumcarbonat, oder tertiäre Amine, beispielsweise Triäthylamin, Pyridin oder auch die entsprechenden Azole selbst im Überschuss verwendet werden. Die Reaktionstemperaturen können zwischen −10 und +150 °C variiert werden, bevorzugt wird der Bereich zwischen 20 bis 120 °C.

Zur Herstellung der N-Halogenmethyl-Verbindungen setzt man beispielsweise auf 1 Mol Schiffsche Base 1 Mol Carbonsäurehalogenid ein. Zur Isolierung der Verbindungen wird beispielsweise entweder filtriert oder das Produkt wird durch Einengen erhalten.

Zur Herstellung der Azol-Verbindungen setzt man auf 1 Mol der N-Halogenmethyl-Verbindung vorzugsweise 1 bis 2 Mol des Azols A–M und 1 Mol Säurebinder ein.

Zur Isolierung der Azol-Verbindungen wird das Reaktionsgemisch beispielsweise filtriert, das Filtrat wird mit Wasser gewaschen, getrocknet und eingeengt. Das so erhaltene Rohprodukt wird gegebenenfalls durch Umkristallisation gereinigt.

Zur Erläuterung sollen folgende chemische Beispiele dienen:

Beispiel 1

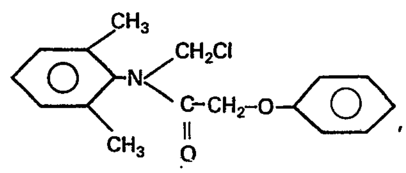

Zu einer Lösung von 34,1 g (0,2 Mol) Phenoxyacetylchlorid in 50 ml Ligroin tropft man langsam bei 0–5 °C eine Lösung von 26,6 g (0,2 Mol) N-Methylen-2,6-dimethylanilin in 100 ml Toluol. Nach 16-stündigem Rühren bei Raumtemperatur wird vom ausgefallenen Produkt abgesaugt. Man isoliert nach dem Trocknen im Vakuum 47 g (78%), N-Chlormethyl- 2,6-dimethyl-phenoxyacetanilid, Fp. 87 °C.

Beispiel 2

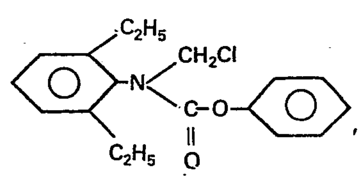

Zu einer Lösung von 31,3 g (0,2 Mol) Chlorameisensäurephenylester in 100 ml Ligroin gibt man bei 15–20 °C unter leichter Kühlung eine Lösung von 32,2 g (0,2 Mol) N-Methylen-2,6-diäthylanilin in 50 ml Ligroin. Nach 16-stündigem Rühren bei Raumtemperatur isoliert man nach Absaugen und Trocknen 49,2 g (76%)

N-Chlormethyl-N-2,6-diäthylphenyl-carbaminsäure-O-phenylester mit Fp. 94–95 °C.

Beispiel 3

315 Raumteile einer Toluol-Lösung, die 1 Mol 2,6-Dimethylphenylazomethin enthält, (Herstellung entsprechend US-PS 3 637 847) wurden unter Rühren bei 5–10 °C zu einer Lösung von 130,5 Gewichtsteilen 2-Furancarbonsäurechlorid in 100 Raumteilen Toluol unter Kühlung zugetropft und anschliessend 10 Stunden bei Raumtemperatur gerührt. Nach dem Kühlen, Absaugen und Trocknen im Vakuum erhielt man 198 Gewichtsteile N-Chlormethylfuran-2-carbonsäure-2',6'-Dimethylanilid vom Schmelzpunkt 124–126 °C.

In entsprechender Weise wurden nachstehende Verbindungen erhalten: Hal=Cl

| Lfd. Nr. | $R^1$ | $R^2$ | $R^3$ | Fp (°C) |
|---|---|---|---|---|
| 1 | $CH_3$ | $CH_3$ | Benzyl | 62–64 |
| 2 | $CH_3$ | $CH_3$ | $CH_2$–S–$CH_3$ | Kristallmasse |
| 3 | $CH_3$ | $C_2H_5$ | do. | 127 |
| 4 | $C_2H_5$ | $C_2H_5$ | do. | Öl |
| 5 | $CH_3$ | $CH_3$ | $CH_2$–O–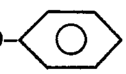 | 87 |
| 6 | $C_2H_5$ | $C_2H_5$ | do. | |
| 7 | $CH_3$ | $CH_3$ | $CH_2$–O–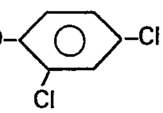 | 122–125 |
| 8 | $C_2H_5$ | $C_2H_5$ | do. | 98–99 |
| 9 | $CH_3$ | $CH_3$ | $CH_2$–O–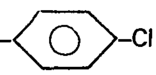 | |
| 10 | $CH_3$ | $CH_3$ | $CH_2$–S–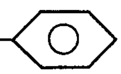 | |
| 11 | $CH_3$ | $CH_3$ | $CH_2$–S–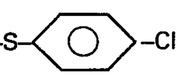 | |
| 12 | $CH_3$ | $CH_3$ | 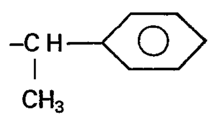 | |
| 13 | $C_2H_5$ | $C_2H_5$ | 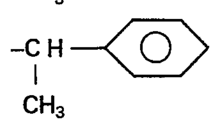 | $n_D^{23}$ = 1,5547 |
| 14 | $C_2H_5$ | $C_2H_5$ | 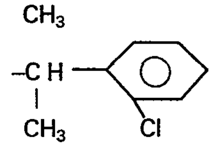 | |
| 15 | $CH_3$ | $CH_3$ | do. | |
| 16 | $CH_3$ | $CH_3$ | Phenyl | 133 |
| 17 | $C_2H_5$ | $C_2H_5$ | Phenyl | 98– 99 |
| 18 | $C_3H_7$–i | $C_3H_7$–i | do. | 107–108 |
| 19 | $CH_3$ | $CH_3$ | 2-Methylphenyl | 118–119 |
| 20 | $CH_3$ | $CH_3$ | 3-Methylphenyl | 133–135 |
| 21 | $CH_3$ | $CH_3$ | 4-Methylphenyl | 119–120 |
| 22 | $CH_3$ | $CH_3$ | 2-Chlorphenyl | 93 |
| 23 | $CH_3$ | $CH_3$ | 3-Chlorphenyl | 145 |
| 24 | $CH_3$ | $CH_3$ | 4-Chlorphenyl | 126 |
| 25 | $CH_3$ | $CH_3$ | 2-Fluorphenyl | 86– 88 |
| 26 | $CH_3$ | $CH_3$ | 3-Fluorphenyl | 114–115 |
| 27 | $CH_3$ | $CH_3$ | 4-Fluorphenyl | 141–143 |
| 28 | $CH_3$ | $CH_3$ | 3-Cyanphenyl | 116–118 |
| 29 | $C_2H_5$ | $C_2H_5$ | 3-Cyanphenyl | 112–114 |
| 30 | $CH_3$ | $CH_3$ | 2-Nitrophenyl | 205–207 |
| 31 | $C_2H_5$ | $C_2H_5$ | 2-Nitrophenyl | |

| Lfd. Nr. | $R^1$ | $R^2$ | $R^3$ | Fp (°C) |
|---|---|---|---|---|
| 32 | $CH_3$ | $CH_3$ | 3-Nitrophenyl | 152–154 |
| 33 | $C_2H_5$ | $C_2H_5$ | 4-Nitrophenyl | 118–120 |
| 34 | $C_2H_5$ | $C_2H_5$ | 3-$CF_3$-phenyl | 63– 64 |
| 35 | $CH_3$ | $CH_3$ | 4-$CF_3$-phenyl | 108–109 |
| 36 | $C_2H_5$ | $C_2H_5$ | 2,4-Dichlorphenyl | 68– 69 |
| 37 | $CH_3$ | $CH_3$ | 2,4-Dichlorphenyl | 108 |
| 38 | $CH_3$ | $CH_3$ | Furyl-2 | 124–126 |
| 39 | $C_2H_5$ | $C_2H_5$ | Furyl-2 | 69– 70 |
| 40 | $CH_3$ | $CH_3$ | Furyl-3 | |
| 41 | $CH_3$ | $CH_3$ | 3,5-Dimethylfuryl | in Lsg. direkt weiterverarb. |
| 42 | $CH_3$ | $CH_3$ | Thienyl-2 | 94– 97 |
| 43 | $CH_3$ | $CH_3$ | Thienyl-3 | in Lsg. direkt weiterverarb. |
| 44 | $CH_3$ | $CH_3$ | 4-Chlorthienyl-3 | 118 |
| 45 | $C_3H_7$-i | $C_3H_7$-i | Thienyl-2 | 94– 95 |
| 46 | $C_3H_7$-i | $C_3H_7$-i | 2,6-Difluorphenyl | 135 |
| 47 | $CH_3$ | $CH_3$ | 3-Methylisoxazolyl-5 | 106 |
| 48 | $CH_3$ | $CH_3$ | 4-Methyl-oxazolyl-5 | 83– 85 |
| 49 | $CH_3$ | $CH_3$ | Phenoxy | 113–115 |
| 50 | $CH_3$ | $CH_3$ | 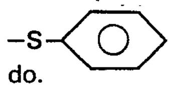 | |
| 51 | $C_2H_5$ | $C_2H_5$ | do. | |
| 52 | $C_2H_5$ | $C_2H_5$ | 2,4-Dichlorphenoxy | |
| 53 | $C_2H_5$ | $C_2H_5$ | 2-Methyl-4-chlor-phenoxy | |
| 54 | $CH_3$ | $C_2H_5$ | 4-Nitrophenoxy | |
| 55 | $C_2H_5$ | $C_2H_5$ | 4-Isopropyl-phenoxy | |
| 56 | $C_2H_5$ | $C_2H_5$ | 4-tert.-Butylphenoxy | |
| 57 | $C_3H_7$-i | $C_3H_7$-i | Phenoxy | 115–116 |

Die neuen N-Halogenmethyl-carbonsäureanilide sind wertvolle Produkte, die sich in einfacher Weise weiterverarbeiten lassen zu wertvollen N-Azolylmethyl-carbonsäureaniliden, die eine überraschende und ähnlichen Verbindungen überraschend überlegene fungizide Wirkung haben.

Die folgenden Beispiele erläutern die Herstellung dieser Verbindungen.

Beispiel 4

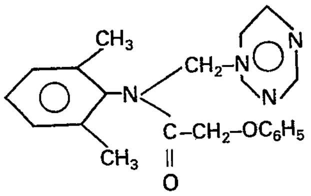

Man suspendiert 10,7 g (155 mMol) 1,2,4-Triazol in 100 ml Toluol und versetzt mit 23,5 g (77,5 mMol) N-Chlormethyl-2,6-dimethyl-(phenoxyacet)-anilid, hergestellt gemäss Beispiel 1. Anschliessend erhitzt man 1 Stunde auf 130 °C, so dass die Reaktionsmischung zum Sieden kommt. Nach dem Abkühlen wird filtriert, mit Toluol gewaschen und das Filtrat eingeengt. Man erhält aus dem Filtrat nach Trocknung 17 g (65% d.Th.) N-1,2,4-Triazolylmethyl-N-phenoxyacetyl-2,6-dimethylanilin mit Fp. 84 °C.

Beispiel 5

20 g (63 mMol) N-Chlormethyl-N-2,6-diäthylphenyl-carbaminsäure-O-phenylester (Beispiel 2) werden in 100 ml Toluol mit 8,6 g (126 mMol) Imidazol gemischt; anschliessend wird die Reaktionsmischung 1 Stunde zum Sieden erhitzt. Das Reaktionsgemisch wird anschliessend noch heiss filtriert. Das nach dem Einengen verbleibende Öl wird nach Anreiben mit Ligroin kristallin. Nach Absaugen und Trocknung erhält man 13,9 g (63% d.Th.) N-Imidazolyl-(1)-methyl-N-2,6-diäthylphenyl-carbaminsäure-O-phenylester vom Schmelzpunkt 115–116 °C.

Beispiel 6

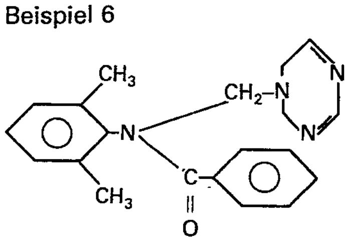

$C_{18}H_{18}N_4O$

a) Man löst 140,5 g (1 Mol) Benzoylchlorid in 200 ml Ligroin und tropft anschliessend bei 5–10 °C eine Lösung von 133 g (1 Mol) N-Methylen-2,6-dimethylanilin in Toluol zu. Nach 14 Stunden Rühren bei 25 °C wird der ausgefallene Feststoff durch Absaugen isoliert. Nach Trocknen im Vakuum erhält man 215 g (79%) N-Chlormethyl-N-benzoyl-2,6-dimethylanilin mit Fp. 133 °C, Formel:

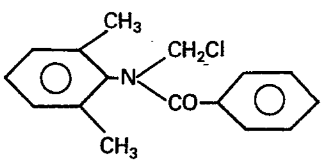

In analoger Weise wurde N-Chlormethyl-N-benzoyl-2,6-diäthylanilin vom Fp. 98–99 °C erhalten.

b) Eine Lösung von 54,7 g (0,2 Mol) N-Chlormethyl-N-benzoyl-2,6-dimethylanilin in 250 ml Tetrahydrofuran wird bei 5–10 °C zu einer Suspension von 13,8 g (0,2 Mol) Triazol in 100 ml Tetrahydrofuran und 20,2 g (0,2 Mol) Triäthylamin zugetropft. Nach 16 Stunden Rühren bei Raumtemperatur (25 °C) wird vom abgeschiedenen Triäthylammoniumhydrochlorid abfiltriert. Nach Abziehen des Lösungsmittels wird der aus dem Filtrat isolierte Feststoff in 250 ml $CH_2Cl_2$ gelöst und mit 100 ml 5-prozentiger (Gew.-%) Salzsäure ausgeschüttelt. Die organische Phase wird nach Trocknung über $Na_2SO_4$ eingeengt; der verbleibende Rückstand wird aus Toluol/Ligroin (3:2) umkristallisiert.

Man erhält 44,6 g (73%) N-(Triazolylmethyl)-N-benzoyl-2,6-dimethylanilin der Formel

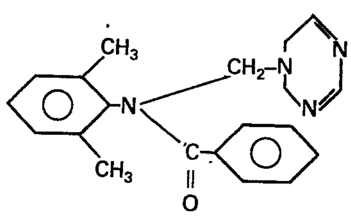

mit Schmelzpunkt 135 °C.

In gleicher Weise wurde N-(Triazolylmethyl)-N-benzoyl-2,6-diäthylanilin hergestellt, Fp. 112–115 °C.

Beispiel 7

a)

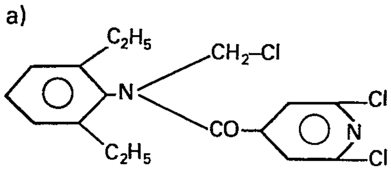

$C_{17}H_{17}Cl_3N_2O$

Zu einer Lösung von 51,4 g (0,24 Mol) 2,6-Dichlor-pyridin-4-carbonsäurechlorid in 250 ml Toluol tropft man 38,6 g (0,24 Mol) N-Methylen-2,6-diäthylanilin. Durch Kühlung mit einem Wasserbad wird die Reaktionstemperatur bei 35–40 °C gehalten. Nach 10-stündigem Rühren wird das Toluol im Vakuum abgezogen (Rotationsverdampfer) und der verbleibende Rückstand mit Ligroin durchgerührt. Man erhält nach Absaugen und Trocknen 67 g (75%) des N-Chlormethylanilids obiger Struktur mit Schmelzpunkt 79–80 °C.

b)

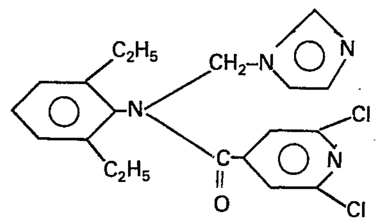

Eine Mischung aus 22,3 g (0,06 Mol) des oben beschriebenen N-Chlormethylanilids und 9 g (0,132 Mol) Imidazol in 100 ml Toluol wird 1 Stunde am Rückflusskühler zum Sieden erhitzt. Anschliessend wird heiss filtriert. Nach Abkühlen des Filtrats isoliert man daraus einen Feststoff, der aus einer 1:1-Mischung von Ligroin und Toluol umkristallisiert wird.

Ausbeute nach Umkristallisation: 11 g (46%) Schmelzpunkt: 120–122 °C.

Die mit Hilfe der neuen N-Halogenmethyl-carbonsäureanilide hergestellten neuen N-Azolylmethyl-carbonsäureanilide zeigen eine starke fungitoxische Wirksamkeit gegen phytopathogene Pilze, insbesondere aus der Klasse der Phycomyceten. Die Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humili an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macropora an Mais, Bremia lactucae an Salat, Mucor mucedo an Früchten, Rhizopus nigricans an Rüben. Die diese Verbindungen enthaltenden fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüberhinaus sind viele der neuen Verbindungen systemisch wirksam, so dass über die Wurzelbehandlung auch ein Schutz oberirdischer Pflanzenteile möglich ist. Die fungizide Wirkung dieser Verbindungen ist der bekannter Fungizide überraschend überlegen.

Ferner lassen sich mit den Verbindungen auch Pilze, die Keimlings- und Auflaufkrankheiten hervorrufen, beispielsweise Pythium- und Aphanomyces-Arten an Leguminosen und Baumwolle, bekämpfen. Die Aufwandmengen betragen je 100 kg Saatgut 10 bis 200 g Wirkstoff; die Anwendung erfolgt in Form von Saatgutbeizmitteln.

**Patentansprüche**
für Vertragsstaaten:
BE, CH, DE, FR, GB, IT, LU, NL, SE

1. Verbindungen der allgemeinen Formel

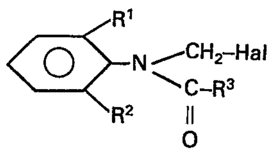

worin
$R^1$ Methyl, Äthyl, Isopropyl, Methoxy oder Chlor,
$R^2$ Methyl, Äthyl, Isopropyl oder Chlor
$R^3$ gegebenenfalls durch Halogen substituiertes Benzyl oder 1-Phenyläthyl,
oder gegebenenfalls ein- oder zweifach durch Methyl, Fluor, Chlor, Brom, Jod, Cyan, Trifluormethyl, Nitro, Methoxy oder Methylthio substituiertes Phenyl,
oder einen gegebenenfalls durch Methyl oder Chlor substituierten 5- oder 6-gliedrigen heterocyclischen Rest mit einem oder zwei gleichen oder verschiedenen Heteroatomen aus der Gruppe Sauerstoff, Schwefel und Stickstoff
oder den Rest $-CH_2-Y$ oder $-\overset{\overset{CH_3}{|}}{C}H-Y$ bedeutet, wobei Y ein $C_1-C_4$-Alkylthiol oder ein gegebenenfalls ein- oder mehrfach durch Chlor, Fluor, Methyl, Trifluormethyl oder Nitro substituiertes Phenoxyl oder Phenylthiol bedeutet,
oder $R^3$ den Rest $-X-R^4$ bedeutet,
wobei X Sauerstoff oder Schwefel,
$R^4$ ein gegebenenfalls ein- oder zweifach durch Fluor, Chlor, Brom, Trifluormethyl, $C_1-C_4$-Alkyl, Nitro, Methoxy oder Methylthio substituiertes Phenyl bedeutet, und Hal Fluor, Chlor oder Brom bedeutet.

2. Verfahren zur Herstellung von N-Halogenmethyl-N-phenylcarbonsäureaniliden der Formel

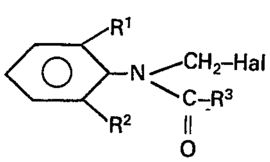

worin
$R^1$ Methyl, Äthyl, Isopropyl, Methoxy oder Chlor,
$R^2$ Methyl, Äthyl, Isopropyl oder Chlor
$R^3$ gegebenenfalls durch Halogen substituiertes Benzyl oder 1-Phenyläthyl,
oder gegebenenfalls ein- oder zweifach durch Methyl, Fluor, Chlor, Brom, Jod, Cyan, Trifluormethyl, Nitro, Methoxy oder Methylthio substituiertes Phenyl,
oder einen gegebenenfalls durch Methyl oder Chlor substituierten 5- oder 6-gliedrigen heterocyclischen Rest mit einem oder zwei gleichen oder verschiedenen Heteroatomen aus der Gruppe Sauerstoff, Schwefel und Stickstoff
oder den Rest $-CH_2-Y$ oder $-\overset{\overset{CH_3}{|}}{C}H-Y$ bedeutet, wobei Y ein $C_1-C_4$-Alkylthiol oder ein gegebenenfalls ein- oder mehrfach durch Chlor, Fluor, Methyl, Trifluormethyl oder Nitro substituiertes Phenoxyl oder Phenylthiol bedeutet,
oder $R^3$ den Rest $-X-R^4$ bedeutet,
wobei X Sauerstoff oder Schwefel,
$R^4$ ein gegebenenfalls ein- oder zweifach durch Fluor, Chlor, Brom, Trifluormethyl, $C_1-C_4$-Alkyl, Nitro, Methoxy oder Methylthio substituiertes Phenyl bedeutet, und Hal Fluor, Chlor oder Brom bedeutet, dadurch gekennzeichnet, dass man ein substituiertes N-Methylenanilin der Formel

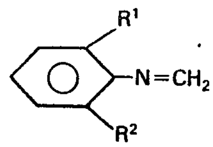

mit einem Carbonsäurehalogenid der Formel $R^3-CO-Hal$ in einem Lösungsmittel umsetzt, wobei Hal, $R^1$, $R^2$ und $R^3$ die in Anspruch 1 genannten Bedeutungen haben.

3. Verfahren zur Herstellung von Verbindungen der Formel

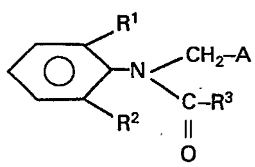

worin
$R^1$ Methyl, Äthyl, Isopropyl, Methoxy oder Chlor,
$R^2$ Methyl, Äthyl, Isopropyl oder Chlor
$R^3$ gegebenenfalls durch Halogen substituiertes Benzyl oder 1-Phenyläthyl,
oder gegebenenfalls ein- oder zweifach durch Methyl, Fluor, Chlor, Brom, Jod, Cyan, Trifluormethyl, Nitro, Methoxy oder Methylthio substituiertes Phenyl, oder einen gegebenenfalls durch Methyl oder Chlor substituierten 5- oder 6-gliedrigen heterocyclischen Rest mit einem oder zwei gleichen oder verschiedenen Heteroatomen aus der Gruppe Sauerstoff, Schwefel und Stickstoff
oder den Rest $-CH_2-Y$ oder $-\overset{\overset{CH_3}{|}}{C}H-Y$ bedeutet, wobei Y ein $C_1-C_4$-Alkylthiol oder ein gegebenenfalls ein- oder mehrfach durch Chlor, Fluor, Methyl, Trifluormethyl oder Nitro substituiertes Phenoxyl oder Phenylthiol bedeutet,
oder $R^3$ den Rest $-X-R^4$ bedeutet,
wobei X Sauerstoff oder Schwefel,
$R^4$ ein gegebenenfalls ein- oder zweifach durch Fluor, Chlor, Brom, Trifluormethyl, $C_1-C_4$-Alkyl, Nitro, Methoxy oder Methylthio substituiertes

Phenyl bedeutet, und Hal Fluor, Chlor oder Brom bedeutet, und

A ein gegebenenfalls ein- oder mehrfach durch Methyl, Äthyl, Isopropyl, Chlor, Brom oder Nitro substituiertes Pyrazol, Imidazol oder 1,2,4-Triazol bedeutet, dadurch gekennzeichnet, daß man ein N-Halogenmethylcarbonsäureanilid der Formel

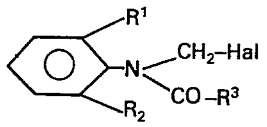

in der $R^1$, $R^2$ und $R^3$ die oben genannten Bedeutungen haben und Hal Fluor, Chlor oder Brom bedeutet, mit einem Azol der Formel A–M, in welcher A die oben angegebene Bedeutung hat und M Wasserstoff, Kalium oder Natrium bedeutet, gegebenenfalls in Gegenwart eines Verdünnungsmittels oder eines Säurebindemittels oder beiden umsetzt.

4. Verbindung gemäss Anspruch 1, nämlich
N-Chlormethylfuran-2-carbonsäure-2',6'-dimethylanilid,
N-Chlormethylthiophen-2-carbonsäure-2',6'-dimethylanilid,
N-Chlormethylthiomethylacet-2,6-dimethylanilid,
N-Chlormethyl-phenoxyacet-2,6-dimethylanilid,
N-Chlormethylphenylcarbonyl-2,6-diethylanilid,
N-Chlormethyl-N-2,6-dimethylphenyl-carbaminsäure-O-phenylester,
N-Chlormethylphenylcarbonyl-2,6-dimethylanilid,
N-Chlormethylphenylacet-2,6-dimethylanilid,
N-Chlormethyl-isoxazol-3-methyl-5-carbonsäure-2,6-dimethylanilid,
N-Chlormethyl-oxazol-4-methyl-5-carbonsäure-2,6-dimethylanilid und
N-Chlormethyl-thiophen-4-chlor-3-carbonsäure-2,6-dimethylanilid.

**Patentansprüche**
für den Vertragsstaat AT

1. Verfahren zur Herstellung von N-Halogenmethyl-N-phenylcarbonsäureaniliden der Formel

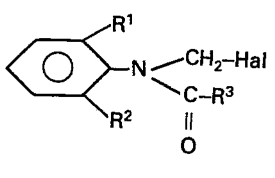

worin

$R^1$ Methyl, Äthyl, Isopropyl, Methoxy oder Chlor,
$R^2$ Methyl, Äthyl, Isopropyl oder Chlor
$R^3$ gegebenenfalls durch Halogen substituiertes Benzyl oder 1-Phenyläthyl,
oder gegebenenfalls ein- oder zweifach durch Methyl, Fluor, Chlor, Brom, Jod, Cyan, Trifluormethyl, Nitro, Methoxy oder Methylthio substituiertes Phenyl, oder einen gegebenenfalls durch Methyl oder Chlor substituierten 5- oder 6-gliedrigen heterocyclischen Rest mit einem oder zwei gleichen oder verschiedenen Heteroatomen aus der Gruppe Sauerstoff, Schwefel und Stickstoff oder den Rest $-CH_2-Y$ oder $-\overset{CH_3}{\overset{|}{C}}H-Y$ bedeutet, wobei Y ein $C_1$–$C_4$-Alkylthiol oder ein gegebenenfalls ein- oder mehrfach durch Chlor, Fluor, Methyl, Trifluormethyl oder Nitro substituiertes Phenoxyl oder Phenylthiol bedeutet,
oder $R^3$ den Rest $-X-R^4$ bedeutet,
wobei X Sauerstoff oder Schwefel,
$R^4$ ein gegebenenfalls ein- oder zweifach durch Fluor, Chlor, Brom, Trifluormethyl, $C_1$–$C_4$-Alkyl, Nitro, Methoxy oder Methylthio substituiertes Phenyl bedeutet,
und Hal Fluor, Chlor oder Brom bedeutet, dadurch gekennzeichnet, dass man ein substituiertes N-Methylenanilin der Formel

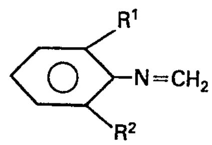

mit einem Carbonsäurehalogenid der Formel $R^3$–CO–Hal in einem Lösungsmittel umsetzt, wobei Hal, $R^1$, $R^2$ und $R^3$ die oben genannten Bedeutungen haben.

2. Verfahren zur Herstellung von Verbindungen der Formel

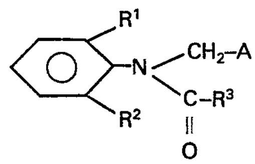

worin

$R^1$ Methyl, Äthyl, Isopropyl, Methoxy oder Chlor,
$R^2$ Methyl, Äthyl, Isopropyl oder Chlor
$R^3$ gegebenenfalls durch Halogen substituiertes Benzyl oder 1-Phenyläthyl,
oder gegebenenfalls ein- oder zweifach durch Methyl, Fluor, Chlor, Brom, Jod, Cyan, Trifluormethyl, Nitro, Methoxy oder Methylthio substituiertes Phenyl, oder einen gegebenenfalls durch Methyl oder Chlor substituierten 5- oder 6-gliedrigen heterocyclischen Rest mit einem oder zwei gleichen oder verschiedenen Heteroatomen aus der Gruppe Sauerstoff, Schwefel und Stickstoff oder den Rest $-CH_2-Y$ oder $-\overset{CH_3}{\overset{|}{C}}H-Y$ bedeutet, wobei Y ein $C_1$–$C_4$-Alkylthiol oder ein gegebenenfalls ein- oder mehrfach durch Chlor, Fluor, Methyl, Trifluormethyl oder Nitro substituiertes Phenoxyl oder Phenylthiol bedeutet,
oder $R^3$ den Rest $-X-R^4$ bedeutet,
wobei X Sauerstoff oder Schwefel,
$R^4$ ein gegebenenfalls ein- oder zweifach durch Fluor, Chlor, Brom, Trifluormethyl, $C_1$–$C_4$-Alkyl, Nitro, Methoxy oder Methylthio substituiertes Phenyl bedeutet, und Hal Fluor, Chlor oder Brom bedeutet und
A ein gegebenenfalls ein- oder mehrfach durch

Methyl, Äthyl, Isopropyl, Chlor, Brom oder Nitro substituiertes Pyrazol, Imidazol oder 1,2,4-Triazol bedeutet, dadurch gekennzeichnet, daß man ein N-Halogenmethylcarbonsäureanilid der Formel

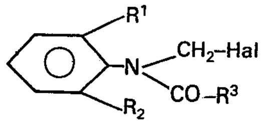

in der $R^1$, $R^2$ und $R^3$ die oben genannten Bedeutungen haben und Hal Fluor, Chlor oder Brom bedeutet, mit einem Azol der Formel A–M, in welcher A die oben angegebene Bedeutung hat und M Wasserstoff, Kalium oder Natrium bedeutet, gegebenenfalls in Gegenwart eines Verdünnungsmittels oder eines Säurebindemittels oder beiden umsetzt.

**Revendications**

pour les Etats contractants:

BE, CH, DE, FR, GB, IT, LU, NL, SE

1. Composés de formule générale

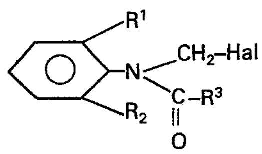

dans laquelle

$R^1$ représente méthyle, éthyle, isopropyle, méthoxy ou chlore,

$R^2$ méthyle, éthyle, isopropyle ou chlore,

$R^3$ benzyle, éventuellement substitué par un halogène ou 1-phényléthyle,

ou phényle, éventuellement substitué une ou deux fois par méthyle, fluor, chlore, brome, iode, cyano, trifluorométhyle, nitro, méthoxy ou méthylthio, ou un reste hétérocyclique à 5 ou 6 membres, éventuellement substitué par méthyle ou chlore avec un ou deux hétéroatomes identiques ou différents appartenant au groupe de l'oxygène, soufre et azote ou

$CH_3$
|
le reste $-CH_2-Y$ ou $-CH-Y$, Y représentant un alkylthiole en $C_1$ à $C_4$ ou un phénoxyle éventuellement substitué une ou deux fois par du chlore, fluor, méthyle, trifluorométhyle, nitro ou du phénylthiole,

ou $R^3$ représente le reste $-X-R^4$,

X représentant l'oxygène ou le soufre,

$R^4$ un phényle éventuellement substitué une ou deux fois par chlore, fluor, brome, trifluorométhyle, alkyle en $C_1$ à $C_4$, nitro, méthoxy ou méthylthio et Hal représentant fluor, chlore ou brome.

2. Procédé pour la préparation d'anilides d'acide N-halogénométhyl-N-phényl-carboxylique de formule

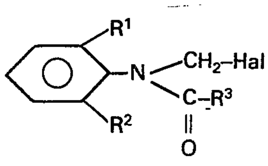

dans laquelle

$R^1$ représente méthyle, éthyle, isopropyle, méthoxy ou chlore,

$R^2$ méthyle, éthyle, isopropyle ou chlore,

$R^3$ benzyle, éventuellement substitué par un halogène ou 1-phényléthyle,

ou phényle, éventuellement substitué une ou deux fois par méthyle, fluor, chlore, brome, iode, cyano, trifluorométhyle, nitro, méthoxy ou méthylthio, ou un reste hétérocyclique à 5 ou 6 membres, éventuellement substitué par méthyle ou chlore avec un ou deux hétéroatomes identiques ou différents appartenant au groupe de l'oxygène, soufre et azote ou

$CH_3$
|
le reste $-CH_2-Y$ ou $-CH-Y$, Y représentant un alkylthiole en $C_1$ à $C_4$ ou un phénoxyle éventuellement substitué une ou deux fois par du chlore, fluor, méthyle, trifluorométhyle, nitro ou du phénylthiole,

ou $R^3$ représente le reste $-X-R^4$,

X représentant l'oxygène ou le soufre,

$R^4$ un phényle éventuellement substitué une ou deux fois par chlore, fluor, brome, trifluorométhyle, alkyle en $C_1$ à $C_4$, nitro, méthoxy ou méthylthio et Hal représentant fluor, chlore ou brome, caractérisé par le fait que l'on fait réagir dans un solvant de la N-méthylènaniline de formule

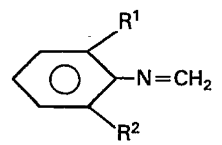

avec un halogénure d'acide carboxylique de formule $R^3-CO-Hal$, Hal, $R^1$, $R^2$ et $R^3$ ayant les significations données dans la revendication 1.

3. Procédé de préparation de composés de formule

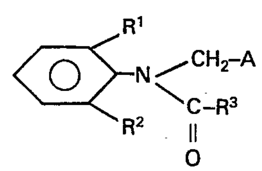

dans laquelle

$R^1$ représente méthyle, éthyle, isopropyle, méthoxy ou chlore,

$R^2$ méthyle, éthyle, isopropyle ou chlore,

$R^3$ benzyle, éventuellement substitué par un halogène ou 1-phényléthyle,

ou phényle, éventuellement substitué une ou deux fois par méthyle, fluor, chlore, brome, iode, cyano, trifluorométhyle, nitro, méthoxy ou méthylthio, ou un reste hétérocyclique à 5 ou 6 membres, éventuellement substitué par méthyle ou chlore avec un ou deux hétéroatomes identiques ou différents appartenant au groupe de l'oxygène, soufre et azote ou

$CH_3$
|
le reste $-CH_2-Y$ ou $-CH-Y$, Y représentant un al-

kylthiole en $C_1$ à $C_4$ ou un phénoxyle éventuellement substitué une ou deux fois par du chlore, fluor, méthyle, trifluorométhyle, nitro ou du phénylthiole,
ou $R^3$ représente le reste $-X-R^4$,
X représentant l'oxygène ou le soufre,
$R^4$ un phényle éventuellement substitué une ou deux fois par chlore, fluor, brome, trifluorométhyle, alkyle en $C_1$ à $C_4$, nitro, méthoxy ou méthylthio et Hal représentant fluor, chlore ou brome, et A représentant un pyrazole, imidazole ou 1,2,4-triazole éventuellement substitué une ou deux fois par méthyle, éthyle, isopropyle, chlore, brome ou nitro, caractérisé par le fait que l'on fait réagir un anilide d'acide N-halogénométhylcarboxylique de formule

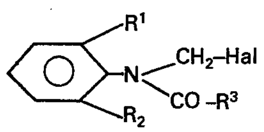

dans laquelle $R^1$, $R^2$ et $R^3$ ont les significations indiquées ci-dessus et Hal représente fluor, chlore ou brome, avec un azole de la formule A–M, dans laquelle A a la signification indiquée ci-dessus et M représente hydrogène, potassium ou sodium, éventuellement en présence d'un diluant ou d'un agent de liaison d'acide ou des deux.

4. Composés selon la revendication 1, à savoir les 2',6'-diméthylanilide d'acide N-chlorométhylfuran-2-carboxylique,
2',6'-diméthylanilide d'acide N-chlorométhylthiophen-2-carboxylique,
N-chlorométhylthiométhylacet-2,6-diméthylanilide,
N-chlorométhyl-phénoxyacet-2,6-diméthylanilide,
N-chlorométhyl-phényl-carbonyl-2,6-diéthylanilide,
O-phénylester d'acide N-chlorométhyl-N-2,6-diméthylphényl carbamique,
N-chlorométhyl-phényl-carbonyl-2,6-diméthylanilide,
N-chlorométhylphénylacet,2,6-diméthylanilide,
2,6-diméthylanilide d'acide N-chlorométhyl-isoxazol-3-méthyl-5-carboxylique,
2,6-diméthylanilide d'acide N-chlorométhyl-oxazol-4-méthyl-5-carboxylique et
2,6-diméthylanilide d'acide N-chlorométhylthiophen-4-chloro-3-carboxylique.

**Revendications**
pour l'Etat contractant AT

1. Procédé pour la préparation d'anilides d'acide N-halogénométhyl-N-phényl-carboxylique de formule

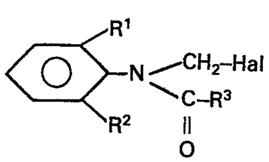

dans laquelle
$R^1$ représente méthyle, éthyle, isopropyle, méthoxy ou chlore,
$R^2$ méthyle, éthyle, isopropyle ou chlore,
$R^3$ benzyle, éventuellement substitué par un halogène ou 1-phényléthyle,
ou phényle, éventuellement substitué une ou deux fois par méthyle, fluor, chlore, brome, iode, cyano, trifluorométhyle, nitro, méthoxy ou méthylthio, ou un reste hétérocyclique à 5 ou 6 membres, éventuellement substitué par méthyle ou chlore avec un ou deux hétéroatomes identiques ou différents appartenant au groupe de l'oxygène, soufre et azote ou

$CH_3$
|
le reste $-CH_2-Y$ ou $-CH-Y$, Y représentant un alkylthiole en $C_1$ à $C_4$ ou un phénoxyle éventuellement substitué une ou deux fois par du chlore, fluor, méthyle, trifluorométhyle, nitro ou du phénylthiole,
ou $R^3$ représente le reste $-X-R^4$,
X représentant l'oxygène ou le soufre,
$R^4$ un phényle éventuellement substitué une ou deux fois par chlore, fluor, brome, trifluorométhyle, alkyle en $C_1$ à $C_4$, nitro, méthoxy ou méthylthio et Hal représentant fluor, chlore ou brome, caractérisé par le fait que l'on fait réagir dans un solvant de la N-méthylènaniline de formule

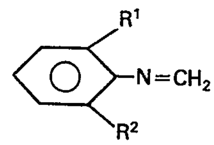

avec un halogénure d'acide carboxylique de formule $R^3-CO-Hal$, Hal, $R^1$, $R^2$ et $R^3$ ayant les significations données dans la présente revendication.

2. Procédé de préparation de composés de formule

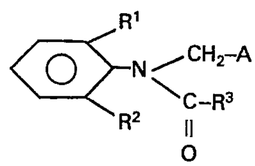

dans laquelle
$R^1$ représente méthyle, éthyle, isopropyle, méthoxy ou chlore,
$R^2$ méthyle, éthyle, isopropyle ou chlore,
$R^3$ benzyle, éventuellement substitué par un halogène ou 1-phényléthyle,
ou phényle, éventuellement substitué une ou deux fois par méthyle, fluor, chlore, brome, iode, cyano, trifluorométhyle, nitro, méthoxy ou méthylthio, ou un reste hétérocyclique à 5 ou 6 membres, éventuellement substitué par méthyle ou chlore avec un ou deux hétéroatomes identiques ou différents appartenant au groupe de l'oxygène, soufre et azote ou

$CH_3$
|
le reste $-CH_2-Y$ ou $-CH-Y$, Y représentant un alkylthiole en $C_1$ à $C_4$ ou un phénoxyle éventuellement substitué une ou deux fois par du chlore,

fluor, méthyle, trifluorométhyle, nitro ou du phénylthiole,
ou $R^3$ représente le reste $-X-R^4$,
X représentant l'oxygène ou le soufre,
$R^4$ un phényle éventuellement substitué une ou deux fois par chlore, fluor, brome, trifluorométhyle, alkyle en $C_1$ à $C_4$, nitro, méthoxy ou méthylthio et Hal représentant fluor, chlore ou brome, et A représentant un pyrazole, imidazole ou 1,2,4-triazole éventuellement substitué une ou deux fois par méthyle, éthyle, isopropyle, chlore, brome ou nitro, caractérisé par le fait que l'on fait réagir un anilide d'acide N-halogénométhylcarboxylique de formule

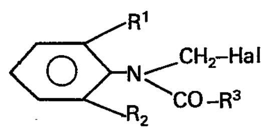

dans laquelle $R^1$, $R^2$ et $R^3$ ont les significations indiquées ci-dessus et Hal représente fluor, chlore ou brome, avec un azole de la formule A–M, dans laquelle A a la signification indiquée ci-dessus et M représente hydrogène, potassium ou sodium, éventuellement en présence d'un diluant ou d'un agent de liaison d'acide ou des deux.

**Claims**
for Contracting States:
BE, CH, DE, FR, GB, IT, LU, NL, SE

1. A compound of the general formula

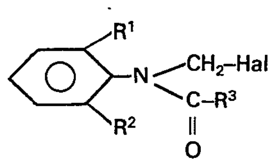

where $R^1$ is methyl, ethyl, isopropyl, methoxy or chlorine, $R^2$ is methyl, ethyl, isopropyl or chlorine, $R^3$ is unsubstituted or halogen-substituted benzyl or 1-phenylethyl, or is phenyl which is unsubstituted or is monosubstituted or disubstituted by methyl, fluorine, chlorine, bromine, iodine, cyano, trifluoro-methyl, nitro, methoxy or methylthio, or is a 5-membered or 6-membered heterocyclic radical which is unsubstituted or substituted by methyl or chlorine and possesses one or two identical or different heteroatoms selected from the group consisting of oxygen,

$CH_3$
|
sulfur and nitrogen, or is $-CH_2-Y$ or $-C$
where Y is $C_1$–$C_4$-alkylthio or is phenoxy or phenylthio which are unsubstituted or are monosubstituted or polysubstituted by chlorine, fluorine, methyl, trifluoromethyl or nitro, or $R^3$ is $-X-R^4$, where X is oxygen or sulfur and $R^4$ is phenyl which is unsubstituted or is monosubstituted or disubstituted by fluorine, chlorine, bromine, trifluoromethyl, $C_1$–$C_4$-alkyl, nitro, methoxy or methylthio, and Hal is fluorine, chlorine or bromine.

2. A process for the preparation of a N-halomethyl-N-phenylcarboxylic acid anilide of the formula

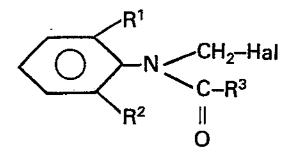

where $R^1$ is methyl, ethyl, isopropyl, methoxy or chlorine, $R^2$ is methyl, ethyl, isopropyl or chlorine, $R^3$ is unsubstituted or halogen-substituted benzyl or 1-phenylethyl, or is phenyl which is unsubstituted or is monosubstituted or disubstituted by methyl, fluorine, chlorine, bromine, iodine, cyano, trifluoromethyl, nitro, methoxy or methylthio, or is a 5-membered or 6-membered heterocyclic radical which is unsubstituted or substituted by methyl or chlorine and possesses one or two identical or different hetero-atoms selected from the group consisting of oxygen, sulfur and nitrogen, or is $-CH_2-Y$

$CH_3$
|
or $-CH-Y$, where Y is $C_1$–$C_4$-alkylthio or is phenoxy or phenylthio which are unsubstituted or are monosubstituted or polysubstituted by chlorine, fluorine, methyl, trifluoromethyl or nitro, or $R^3$ is $-X-R^4$, where X is oxygen or sulfur and $R^4$ is phenyl which is unsubstituted or is monosubstituted or disubstituted by fluorine, chlorine, bromine, trifluoromethyl, $C_1$–$C_4$-alkyl, nitro, methoxy or methylthio, and Hal is fluorine, chlorine or bromine, characterized in that a substituted N-methylene-aniline of the formula

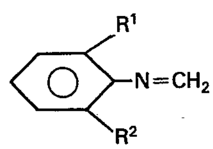

is reacted with a carboxylic acid halide of the formula $R^3$–CO–Hal in a solvent, Hal, $R^1$, $R^2$ and $R^3$ having the meanings fiven in claim 1.

3. A process for the preparation of a compound of the formula

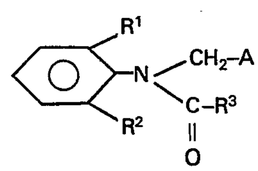

where $R^1$ is methyl, ethyl, isopropyl, methoxy or chlorine, $R^2$ is methyl, ethyl, isopropyl or chlorine, $R^3$ is unsubstituted or halogen-substituted benzyl or 1-phenylethyl, or is phenyl which is unsubstituted or is monosubstituted or disubstituted by methyl, fluorine, chlorine, bromine, iodine, cyano, trifluoromethyl, nitro methoxy or methylthio, or is a 5-membered or 6-membered heterocyclic radical which is unsubstituted or substituted by methyl or chlorine and possesses one or two identical or different hetero-atoms selected from

the group consisting of oxygen, sulfur and nitrogen, or

$CH_3$
|
$-CH_2-Y$ or $-CH-Y$, where Y is $C_1$–$C_4$-alkylthio or is phenoxy or phenylthio which are unsubstituted or are monosubstituted or polysubstituted by chlorine, fluorine, methyl, trifluoromethyl or nitro, or $R^3$ is $-X-R^4$, where X is oxygen or sulfur and $R^4$ is phenyl which is unsubstituted or is monosubstituted or disubstituted by fluorine, chlorine, bromine, trifluoromethyl, $C_1$–$C_4$-alkyl, nitro, methoxy or methylthio, and A is a pyrazole, imidazole or 1,2,4-triazole radical, each of which is unsubstituted or is monosubstituted or polysubstituted by methyl, ethyl, isopropyl, chlorine, bromine or nitro, characterized in that a N-halomethylcarboxylic acid anilide of the formula

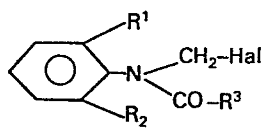

where $R^1$, $R^2$ and $R^3$ have the above meanings and Hal is fluorine, chlorine or bromine, is reacted with an azole of the formula A–M, where A has the above meanings and M is hydrogen, potassium or sodium, in the presence or absence of a diluent or of an acid acceptor or of both.

4. A compound as claimed in claim 1, namely N-chloromethylfuran-2-carboxylic acid-2',6'-dimethylanilide,
N-chloromethylthiophene-2-carboxylic acid-2',6'-dimethylanilide,
N-chloromethylthiomethylacet-2,6-dimethylanilide,
N-chloromethyl-phenoxyacet-2,6-dimethylanilide,
N-chloromethylphenylcarbonyl-2,6-diethylanilide,
N-chloromethyl-N-2,6-dimethylphenyl-carbamic acid-O-phenylester,
N-chloromethylphenylcarbonyl-2,6-dimethylanilide,
N-chloromethylphenylacet-2,6-dimethyl-anilide,
N-chloromethylisoxazole-3-methyl-5-carboxylic acid-2,6-dimethyl-anilide,
N-chloromethyl-oxazole-4-methyl-5-carboxylic acid-2,6-dimethylanilide or
N-chloromethyl-thiophene-4-chloro-3-carbocylic acid-2,6-dimethylanilide.

**Claims** for AT

1. A process for the preparation of a N-halomethyl-N-phenylcarboxylic acid anilide of the formula

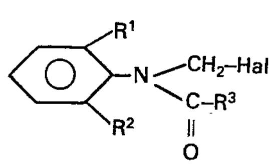

where $R^1$ is methyl, ethyl, isopropyl, methoxy or chlorine, $R^2$ is methyl, ethyl, isopropyl or chlorine, $R^3$ is unsubstituted or halogen-substituted benzyl or 1-phenylethyl, or is phenyl which is unsubstituted or is monosubstituted or disubstituted by methyl, fluorine, chlorine, bromine, iodine, cyano, trifluoromethyl, nitro, methoxy or methylthio, or is a 5-membered or 6-membered heterocyclic radical which is unsubstituted or substituted by methyl or chlorine and possesses one or two identical or different hetero-atoms selected from the group consisting of oxygen,

$CH_3$
|
sulfur and nitrogen, or is $-CH_2-Y$ or $-CH-Y$, where Y is $C_1$–$C_4$-alkylthio or is phenoxy or phenylthio which are unsubstituted or are monosubstituted or polysubstituted by chlorine, fluorine, methyl, trifluoromethyl or nitro, or $R^3$ is $-X-R^4$, where X is oxygen or sulfur and $R^4$ is phenyl which is unsubstituted or is monosubstituted or disubstituted by fluorine, chlorine, bromine, trifluoromethyl, $C_1$–$C_4$-alkyl, nitro, methoxy or methylthio, and Hal is fluorine, chlorine or bromine, characterized in that a substituted N-methylene-aniline of the formula

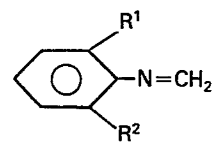

is reacted with a carboxylic acid halide of the formula $R^3$–CO–Hal in a solvent, Hal, $R^1$, $R^2$ and $R^3$ having the avobe-mentioned meanings.

2. A process for the preparation of a compound of the formula

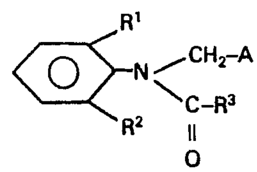

where $R^1$ is methyl, ethyl, isopropyl, methoxy or chlorine, $R^2$ is methyl, ethyl, isopropyl or chlorine, $R^3$ is unsubstituted or halogen-substituted benzyl or 1-phenylethyl, or is phenyl which is unsubstituted or is monosubstituted or disubstituted by methyl, fluorine, chlorine, bromine, iodine, cyano, trifluoromethyl, nitro, methoxy or methylthio, or is a 5-membered or 6-membered heterocyclic radical which is unsubstituted or substituted by methyl or chlorine and possesses one or two identical or different hetero-atoms selected from the group consisting of oxygen,

$CH_3$
|
sulfur and nitrogen, or is $-CH_2-Y$ or $-CH-Y$, where Y is $C_1$–$C_4$-alkylthio or is phenoxy or phenylthio which are unsubstituted or are monosubstituted or polysubstituted by chlorine, fluorine, methyl, trifluoromethyl or nitro, or $R^3$ is $-X-R^4$, where X is oxygen or sulfur and $R^4$ is phenyl which is unsubstituted or is monosubstituted-

tuted or disubstituted by fluorine, chlorine, bromine, trifluoromethyl, $C_1$–$C_4$-alkyl, nitro, methoxy or methylthio, and A is a pyrazole, imidazole or 1,2,4-triazole radical, each of which is unsubstituted or is monosubstituted or polysubstituted by methyl, ethyl, isopropyl, chlorine, bromine or nitro, characterized in that a N-halomethylcarboxylic acid anilide of the formula

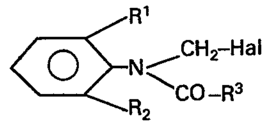

where $R^1$, $R^2$ and $R^3$ have the above meanings and Hal is fluorine, chlorine or bromine, is reacted with an azole of the formula A–M, where A has the above meanings and M is hydrogen, potassium or sodium in the presence or absence of a diluent or of an acid acceptor or of both.